# EUROPEAN PATENT SPECIFICATION

(11) **EP 2 655 594 B1**
(45) Date of publication and mention of the grant of the patent: **15.02.2017**
(21) Application number: 11799436.8
(22) Date of filing: 20.12.2011
(51) Int. Cl.: C12N 1/20, C12P 7/26, A23C 9/12, C12P 7/24, C12P 7/04, C12R 1/46

(54) **FLAVOUR MODULATION BY BIO-PROCESSING USING FLAVOUR FORMING BACTERIA STRAINS**
GESCHMACKSMODULATION DURCH BIOVERARBEITUNG ANHAND VON GESCHMACKBILDENDEN BAKTERIENSTÄMMEN
MODULATION DE SAVEUR PAR TRAITEMENT BIOLOGIQUE AU MOYEN DE SOUCHES DE BACTÉRIES FORMANT DES SAVEURS

(30) Priority: 20.12.2010 EP 10195845
(43) Date of publication of application: 30.10.2013
(73) Proprietor: Nestec S.A., 1800 Vevey (CH)
(72) Inventor: BRAUN, Marcel, 3510 Konolfingen (CH)
(74) Representative: Künzi, Sophie
(86) International application number: PCT/EP2011/073489
(87) International publication number: WO 2012/085009

(56) References cited:
- WO-A1-99/33351
- WO-A1-2008/049581
- MUTUKUMIRA A N ET AL: "Characterisation of a malty-compound producing Lactococcus lactis subsp lactis biovar. diacetylactis C1 strain isolated from naturally fermented milk", MILCHWISSENSCHAFT, vol. 64, no. 1, 2009, pages 26-29, XP009147172, ISSN: 0026-3788
- NARVHUS J A ET AL: "Production of fermented milk using a malty compound-producing strain of Lactococcus lactis subsp. lactis biovar. diacetylactis, isolated from Zimbabwean naturally fermented milk", INTERNATIONAL JOURNAL OF FOOD MICROBIOLOGY, vol. 41, no. 1, 5 May 1998 (1998-05-05), pages 73-80, XP002637587, ISSN: 0168-1605
- BOUMERDASSI H ET AL: "Effect of Citrate on Production of Diacetyl and Acetoin by Lactococcus lactis ssp. lactis CNRZ 483 Cultivated in the Presence of Oxygen", JOURNAL OF DAIRY SCIENCE, AMERICAN DAIRY SCIENCE ASSOCIATION, US, vol. 80, no. 4, 1 April 1997 (1997-04-01), pages 634-639, XP027047845, ISSN: 0022-0302 [retrieved on 1997-04-01]
- HUGENHOLTZ J ET AL: "DIACETYL PRODUCTION BY DIFFERENT STRAINS OF LACTOCOCCUS LACTIS SUBSP. LACTIS VAR. DIACETYLACTIS AND LEUKONOSTOC SPP", APPLIED MICROBIOLOGY AND BIOTECHNOLOGY, SPRINGER VERLAG, BERLIN, DE, vol. 38, 1 January 1992 (1992-01-01), pages 17-22, XP000606685, ISSN: 0175-7598, DOI: DOI:10.1007/BF00169412

## Description

### Field of invention

The present invention relates to the generation of flavour and aroma in milk-based products. The generation of flavour and aroma in milk-based products is achieved using bacteria strains during fermentation of a milk source.

### Background of the invention

Fermentation is a conversion of carbohydrates to organic acids or other compounds using bacteria strains.

Fermented milk products are major consumer products. Fermented milk products can be, for example, cheeses, buttermilks and yoghurts. Fermented milk products are manufactured by fermenting a milk source.

A milk source, for example milk, contains the carbohydrate lactose. During fermentation of the milk source the bacteria strains ferment the carbohydrate lactose to produce lactic acid. The production of lactic acid results in an acidification of the milk source during the manufacture of the fermented milk product. During fermentation of the milk source, other reactions may occur between other substances present in the milk source and the bacterial strains.

A fermentation of the milk source with bacteria strains is responsible for a generation of a flavour and aroma in the fermented milk products. Furthermore the fermentation of the milk source with the bacteria strains increases a shelf-life of the fermented milk products.

The bacteria strains used to ferment the milk source can be lactic acid bacteria strains. The lactic acid bacteria strains include Lactobacillus, Leuconostoc, Pediococcus, Lactococcus and Streptococcus; as well as the more peripheral Aerococcus, Carnobacterium, Enterococcus, Oenococcus, Sporolactobacillus, Teragenococcus, Vagococcus and Weisella; these lactic acid bacteria strains belong to the order Lactobacillales.

An international patent application publication No. WO 2008/049581 by the Applicant Nestec SA is titled "Taste and flavour modulation by biotransformation in milk products". The international patent application publication No. WO 2008/049581 discloses a method to promote a non-savoury flavour in a food product.

An international patent application publication No. WO 02/085131 by the Applicant New Zealand Dairy Board is titled "Method of preparing savoury-flavoured products by fermentation of proteins". The international patent application publication No. 02/085131 discloses a method for the manufacture of a savoury flavoured product from a source of protein using a combination of two distinct strains of bacteria. The source of protein may be a plant soy, wheat, rice, milk or whey. A first strain of bacteria is selected from the group Macrococcus, Micrococcus, Entercoccus, Staphylococcus, Brevibacterium, Anthrobacter and Corynebacterium, preferably Macrococcus caseolyticus. A second strain of bacteria is selected from the lactic acid bacteria - Lactococcus, Lactobacillus, Pediococcus or Leuconostoc. The savoury flavoured product may be combined with other ingredients to form products such as cheese, protein-water gels, yoghurts, creams, custards, sauces and confectionary products.

An international patent application publication No. WO 02/00845 by the Applicant Nizo Food Research is titled "Enhanced flavour production in or relating to food by cultivation of various food grade micro-organisms". The international patent application publication No. WO 02/00845 discloses new mixed cultures of two or more micro-organism strains wherein at least one of the micro-organism strains which are comprised in said mixed culture is individually selected on the basis of its ability to perform part of an enzymatic pathway, and said two or more selected micro-organism strains together form a complete pathway towards a desired flavour component. The mixed culture is a culture for the production of a fermented product, such as yogurt or cheese or sausage. Said two or more micro-organism strains are preferably co-cultivated. Particular and preferred embodiments are starter cultures for the manufacture of cheese. The mixed culture comprising a combination of various Lactoccocus strains and a combination of a Brevibacterium strain and a Staphylococcus strain, respectively.

The article "Characterisation of a malty-compound producing Lactococcus lactis subsp. lactis biovar. diacetylactis C1 strain isolated from naturally fermented milk" by Mutukumira et al. (2009) Milchwissenschaft 64(1) pp. 26-29, relates to a strain that produced acceptable fermented milk to a sensory panel despite the presence of a slight malty flavour.

The article "Production of fermented milk using a malty compound-producing strain of *Lactococcus lactis* subsp. *lactis* biovar. *diacetylactis,* isolated from Zimbabwean naturally fermented milk" by Narvhus et al. (1998) Internat. J. Food Microbiol. 41, pp. 73-80, relates to the preparation of fermented milk from several milks, using strain a strain of *Lactococcus lactis* subsp. *lactis* biovar. *diacetylactis* isolated from Zimbabwean naturally fermented milk.

However, due to a number of the lactic acid bacteria strains and their interactions with individuals, a selection of certain lactic acid bacteria strains to produce certain flavours and aromas in the fermented milk products is not predictable.

Fermented milk products have a wide variety of flavours and aromas depending upon the milk source and the lactic acid bacteria strains used to ferment the milk source.

There is a need to provide methods and lactic acid bacteria strains that are responsible for specific flavours and aromas in the fermented milk products.

Furthermore, artificial additives are negatively perceived by the consumer. There a need to provide flavour and aromas in a natural way that avoids artificial additives.

There is also a need to provide flavour and aromas which can be used in a wide range of foods.

There is thus a need to overcome the aforementioned problems in the art.

### Summary of invention

In an aspect the present invention relates to a method for the manufacture of a fermented milk product. The fermented milk product has at least a malty flavour and aroma. The method comprises providing a milk source, forming an amino acid supplemented milk source, to the amino acid supplemented milk source is added a Lactococcus lactis subsp. lactis biovar diacetylactis (CNCM No. I-1962) to form a mixture. The mixture is then fermented to manufacture the fermented milk product.

In a further aspect the present invention relates to a fermented milk product with at least a malty flavour and aroma obtained by the aforementioned method.

In a further aspect the present invention relates to a product for consumption by a mammal comprising the fermented milk product with at least a malty flavour and aroma.

In a further aspect the present invention relates to a food product comprising a lactic acid bacterium, Lactococcus lactis subsp. lactis biovar diacetylactis (CNCM No. I-1962) any one of 2-methylpropanal, 2/3-methylbutanal, phenylacetaldehyde and 2/3-methylbutanol.

In a further aspect the present invention relates to a use of a lactic acid bacterium, Lactococcus lactis subsp. lactis biovar diacetylactis (CNCM No. I-1962) to impart at least at least a malty flavour and aroma to a milk source.

The present inventors were surprised to find that a lactic acid bacterium, Lactococcus lactis subsp. lactis biovar diacetylactis (CNCM No. I-1962) imparts such flavour and aroma to the fermented milk products.

### Detailed description of invention

For a complete understanding of the present invention and the advantages thereof, reference is made to the following detailed description of the invention.

It should be appreciated that various aspects of the present invention are merely illustrative of the specific ways to make and use the present invention.

The various aspects of the present invention can be combined with other aspects of the present invention and do not limit the scope of the invention when taken into consideration with the claims and the following detailed description.

The present invention concerns fermented milk products. The fermented milk products are manufactured by a fermentation of a milk source with a lactic acid bacterium to provide flavour and aroma to the fermented milk products.

The lactic acid bacterium is Lactococcus lactis subsp. lactis biovar diacetylactis. The lactic acid bacterium, Lactococcus lactis subsp. lactis biovar diacetylactis NCC 2415 was deposited in 1997 with the Institut Pasteur - Collection Nationale de Cultures de Mico-organisme (CNCM) with a CNCM No. I-1962.

The milk source can be any type of milk, such as cow milk, sheep milk, goat milk and buffalo milk or any mixtures thereof. The milk source may be UHT-treated milk, pasteurised milk or non-pasteurised milk. The milk source may be full fat milk, a skimmed milk or semi-skimmed milk. Furthermore the milk source may be a fresh milk, recombined milk and milk containing vegetable fat and any mixtures thereof.

A conversion of amino acids into volatile flavour and aroma compounds plays an important role in food technology. The conversion of amino acids into volatile flavour and aroma compounds can be achieved by the lactic acid bacterium. Therefore there is a need to supplement the milk source with amino acids. That is to say that amino acids are supplemented to the milk source in addition to any amino acids that are naturally present in the milk source. In order to supplement the milk source at least one of an amino acid, a protease or a peptidase or any mixture thereof is added to the milk source. The amino acids are at least one of L-phenylalanine, L-leucine, L-isoleucine, L-valine. It is preferable that the peptidases is an exo-peptidases applied as enzyme preparation (for example produced by Aspergillus oryzae, Aspergillus sojae, Rhizophus oryzae, Bacillus subtilis, Ananas comosus) or as microbial strains with exoproteolytic activity (for example L. helveticus, S. thermophilus, L. plantarum, L. lactis subspecies). The peptidases or proteases release amino acid(s) such as L-phenylalanine, L-leucine, L-isoleucine, L-valine by an interaction with peptides and proteins naturally present in the milk source.

The amino acids are converted into volatile flavour and aroma compounds which are honey-like, malt-like or chocolate-like volatile flavour and aroma compounds.

The peptidases or proteases can be added without the amino acid(s) or with the amino acids. The at least one of the amino acid, the protease or the peptidase or any mixture thereof are added to the milk source in amount of 0.01 to 5 wt%, preferably 0.01-2 wt%, more preferably in an amount of 0.03-1.0 wt%, most preferably 0.05-0.3 wt%.

To the supplemented milk source is added the lactic acid bacterium, Lactococcus lactis subsp. lactis biovar diacetylactis (CNCM No. I-1962) to form a mixture.

Lipase can also be added to the milk source or the mixture to produce a desired flavour and aroma in the fermented milk products. Lipase hydrolyses fats in the milk source to form for example di-glycerides, monoglycerides and free fatty acids or any mixtures thereof. The di-glycerides, monoglycerides and free fatty acids impart a cream-like flavour to the fermented milk product. Lactase can also be added to the milk source or the mixture to produce desired flavour and aroma in the fermented milk products. Lactase hydrolyses the disaccharide lactose in the milk source into galactose and glucose. Glucose and galactose are used as flavour precursors for caramel-like and sweetened condensed milk-like flavour formation.

If the milk source is non-pasteurised, the milk source or the supplemented milk source may be pasteurised, subjected to ultra-high temperature treatment (UHT-milk) or sterilised under conditions known in the art. The pasteurisation, ultra-high temperature treatment and sterilisation is carried out in a temperature range of 70°C to 150°C for a time of between 2s to 20min. Alternatively, the milk source may be heat-treated prior to being the supplemented milk source.

The mixture of the supplemented milk source and the lactic acid bacterium (Lactococcus lactis subsp. lactis biovar diacetylactis) is then fermented to manufacture the fermented milk product with the malty flavour and aroma. The fermentation is allowed to take place for between 6 and 24 hours at a temperature of approximately 30°C. Optionally, fermentation improving cofactors such as alpha-ketoglutarate, manganese or magnesium salts may also be added prior to the fermentation.

Depending upon the milk source it is to be appreciated that the fermented milk product with a malty flavour and aroma can be in the form of slurry (yogurt like) or a liquid. The fermented milk product can be further dried or concentrated.

The fermented milk product with the malty flavour and aroma can be dried, preferably by spray-drying and then converted into a powder.

Fermented milk product with the malty flavour and aroma can have applications in food products and during a manufacture of food products. For example, the powder with the malty flavour and aroma can have applications in the beverage industry to impart the malty flavour and aroma to beverages. For example, the powder with the malty flavour and aroma can have applications in the food industry to impart the malty flavour and aroma to foodstuffs.

### Examples

The manufactured fermented milk products were analysed by an electronic nose based on mass spectrometry and gas chromatography coupled to mass spectrometry (GC-MS).

Analysis with electronic nose based on mass spectrometry is a direct analysis method wherein the fermented milk product is placed directly into the ion source without the need for separation procedures and is therefore timesaving. A determination of volatiles from such a resultant mass spectra contains limited information for the identification of aroma components. Unequivocal identification of the single compounds present is not possible without prior separation and selective fragmentation i.e. GC-MS.

Gas chromatography coupled to mass spectrometry (GC-MS) provides the necessary separation and detection of volatiles. GC-MS is used for obtaining MS fragments belonging to a specific aroma component. The unambiguous identification of the molecules by GC-MS in combination with olfactometry is mandatory for analysing volatiles with a specific odour.

Commonly used extraction methods for the isolation of volatiles from fermented milk products are vacuum distillation followed by solvent extraction, purge and trap (PT) and headspace techniques such as headspace solid-phase micro extraction (HS-SPME). The purge and trap (PT) and headspace techniques methods identify volatiles with different yield performances, but with comparable reproducibility. PT appeared to be a more sensitive whereas SPME is a more rapid and less expensive technique.

The reagents where used, were used as received without prior treatment unless otherwise stated.

### Example 1

### A - Reactivation of lactic acid bacterium

The lactic acid bacterium, Lactococcus lactis subsp. lactis biovar diacetylactis (CNCM No. I-1962) in ampoules was reactivated with 1 ml reconstituted milk under sterile conditions, transferred in sterile glass tubes containing 9 ml reconstituted milk and incubated aerobically at 30°C for 24 h in the dark.

The lactic acid bacterium, Lactococcus lactis subsp. lactis biovar diacetylactis (CNCM No. I-1962) were stored at 6°C for two weeks and subsequently inoculated at 0.5% ((*v*/*v*) 0.05/10 ml medium) in a culture.

The culture was M17x (M17 Terzaghi Bouillon, Merck 1.15029 and 5g/l glucose (Merck 8342). After the growth phase (3 days) the flasks were stored at 6°C to form a reactivated lactic acid bacterium.

Alternatively the culture can be skimmed milk.

### B - Milk source supplementation with amino acids

An amino acid solution of 100 mM L-phenylalanine (Fluka, Buchs, Switzerland) (1.65g/100ml), L-leucine (Merck, Darmstadt, Germany) (1.31g/100ml), L-isoleucine (Merck, Darmstadt, Germany) (1.31g/100ml), L-valine (Merck, Darmstadt, Germany) (1.17g/100ml) was dissolved in sterile water. The amino acid solution was filtrated through a pore size of 0.45µm (Schleicher & Schuell, Whatmann, FP 30/0.45µm, 7bar max. CA-S). 500 µl of the amino acid solution was added to 4.5 ml UHT-milk (dilution 1:10) to obtain an supplemented milk source with a final concentration of 10mM in 5.0ml.

### C - Fermentation

Fermentation in UHT-milk was performed by two approaches (**I** - **II**).
**I:** Index (Inside needle dynamic extraction; Hamilton) headspace sampling of volatile compound fragments in non-supplemented UHT-milk.
**II:** Tenax (accumulation adsorbens, Marin-Epagnier, Switzerland) headspace sampling of volatile compounds in supplemented milk source UHT-milk (10 mM L-leucine, L-isoleucine, L-valine and L-phenylalanine).

An aliquot of 50 µl of the reactivated lactic acid bacterium was transferred in 5 ml supplemented milk source UHT milk (1% inoculation) under sterile conditions and incubated at 30°C aerobically for 16-24 hours in the dark. An addition of 2.8g NaCl into the headspace vials helped to expel the volatiles from the fermented milk product into the headspace to get more intense release of the volatiles.

A electronic nose detected the volatile compound fragments at a range of *m*/*z* 40 - 100 for the experiment with non-supplemented UHT-milk (i.e. no amino acids) and at *m*/*z* 10 - 160 for the experiment with supplemented UHT-milk (i.e. with amino acids).

Principle component analysis (PCA) was calculated using the software program "The Unscrambler" (version 9.7). The results were calculated with logarithmised raw data and exclusion of the water and milk blanks. The calculations were done with all variables (MS fragments) included to group the strains in relation to similar MS-fragment patterns and abundance of compounds.

### D - Electronic nose measurements

Analysis of the ferments milk product by the electronic nose measurements in supplemented milk source UHT-milk was conducted. II: Tenax headspace measurement with 10mM Leu, Ile, Val and Phe supplemented UHT-milk. GC-MS fragments [M]⁺ were 27, 29, 43, 45, 60, 70, 86, 87, 88, 91, 103 and 120.

### E - pH and redox potential

A ph of the fermented milk product was determined to be 4.3 with a redox potential of 50mV.

### F - Sensory assessment of fermented milk product

After fermentation the glass vials were kept close until sensory evaluation started. Seven persons attended the sensory assessment of the fermented milk product. The sensory assessment was based on the following attributes, scoring is noted with a X. A blank sample (incubated milk) was given as a reference. In order to test the influence of the amino acids samples were also prepared without the amino acids (addition of sterile water only) and presented to the panel. The results are shown below, wherein an X indicated a sensory perception of the fermented milk product.

| | |
|---|---|
| Buttery | - |
| Bitter/-almond | XX |
| Flower-like | X |
| Bread-like | **XXXX** |
| Creamy | X |
| Caramel | - |
| Strawberry | - |
| Fresh | - |
| Fruity | - |
| Yeast | X |
| Honey | X |
| Yoghurt | - |
| Cheesy | - |
| Milky | - |
| Malty | **XXXX** |
| Almond | XX |
| Nutty | X |
| Paper-like | - |
| Sweet | X |
| Acidic | X |
| Salty | - |
| Vanilla | - |

The results of the sensory assessment of fermented milk product demonstrate that the fermented milk product had a malty bread like flavour aroma. Other experiments have shown that by selection of the amino acid(s) different flavours and aromas can be generated.

### Example 2

### A - Reactivation of lactic acid bacterium

A reactivation of lactic acid bacterium was carried out according to Example 1.

### B - Milk source supplementation with amino acids

An amino acid solution L-leucine (Merck, Darmstadt, Germany) (1.31g/100ml), L-isoleucine (Merck, Darmstadt, Germany) (1.31g/100ml), L-phenylalanine (Fluka, Buchs, Switzerland) (1.65g/100ml) was dissolved in sterile water. The amino acid solution was filtrated through a pore size of 0.45µm (Schleicher & Schuell, Whatmann, FP 30/0.45µm, 7bar max. CA-S). 500 µl of the amino acid solution was added to 4.5 ml UHT-milk (dilution 1:10) to obtain an supplemented milk source with a final concentration of 10mM in 5.0ml.

### C - Fermentation

Fermentation in UHT-milk was performed by two approaches (**I** - **II**).
**I:** Index (Inside needle dynamic extraction; Hamilton) headspace sampling of volatile compound fragments in non-supplemented UHT-milk.
**II:** Tenax (accumulation adsorbens, Marin-Epagnier, Switzerland) headspace sampling of volatile compounds in supplemented UHT-milk (10 mM L-leucine, L-isoleucine, L-valine and L-phenylalanine).

An aliquot of 500 µl of the reactivated lactic acid bacterium was transferred in 5 ml supplemented milk source UHT milk (2% inoculation) under sterile conditions and incubated at 30°C aerobically for 16 hours in the dark. An addition of 2.8g NaCl into the headspace vials helped to expel the volatiles from the fermented milk product into the headspace to get more intense release of the volatiles.

The electronic nose detected the volatile compound fragments at a range of *m*/*z* 40 - 100 for the experiment with non-supplemented UHT-milk (i.e. no amino acids) and at *m*/*z* 10 - 160 for the experiment with supplemented UHT-milk (i.e. with amino acids).

Principle component analysis (PCA) was calculated using the software program "The Unscrambler" (version 9.7). The results were calculated with logarithmised raw data and exclusion of the water and milk blanks. The calculations were done with all variables (MS fragments) included to group the strains in relation to similar MS-fragment patterns and abundance of compounds.

### D - Electronic nose measurements

Analysis of the ferments milk product by the electronic nose measurements in supplemented UHT-milk was conducted. II: Tenax headspace measurement with 10mM Leu, Ile, Val and Phe supplemented UHT-milk. GC-MS fragments [M]⁺ were 27, 29, 43, 45, 60, 70, 86, 87, 88, 91, 103 and 120.

### E - pH and redox potential

A ph of the fermented milk product was determined to be 4.78.

### F - Sensory assessment of fermented milk product

After bacterial fermentation the glass vials were kept close until sensory evaluation started. Ten persons attended the sensory assessment of the fermented milk product. The sensory evaluation was a taste evaluation in order to gain information on the in mouth - effect and taste of the obtained fermented milk products.

In each case the samples were pasteurised (85 °C for 15 min in a water bath) and diluted to 1 % in UHT milk (at a temperature 20-25°C). The results shown below detail the inferences of the panellists.

| Panellist | Blank Incubated UHT-milk | Fermented milk product |
|---|---|---|
| 1 | Slightly milky | Malty, Ovomaltine |
| 2 | Milky, UHT-milk Slightly Sour | Strong malty, Ovomaltine-like, sweetish, slightly chocolate-like |
| 3 | Milky, Slight almond, Bitter | Yeasty |
| 4 | Milky | Malted |
| 5 | Milky, Cooked | Malted |
| 6 | Milk powder, Sweet | Malt with honey note |
| 7 | Milky, Fatty, Buttery, Sweet | Malty cereals |
| 8 | - | Malty |
| 9 | - | Malty, Honey |
| 10 | - | Old, Stale. |

The results of the sensory assessment of fermented milk product demonstrate that the fermented milk product had a predominantly malty like flavour aroma with a twist of honey-chocolate like flavour aroma.

The malty note can be attributed to the presence of 2-methylpropanal, 2/3-methylbutanal, phenylacetaldehyde and 2/3-methylbutanol as determined by the electronic nose measurements.

Having thus described the present invention in detail, it is to be understood that the detailed description is not intended to limit the scope of the invention thereof.

What is desired to be protected by letters patent is set forth in the following claims.

## Claims

1. A method for the manufacture of a fermented milk product with at least a malty flavour and aroma comprising:
- providing a milk source,
- forming an amino acid supplemented milk source,
- adding to the amino acid supplemented milk source a Lactococcus lactis subsp. lactis biovar diacetylactis, deposited as CNCM I-1962, to form a mixture; and
- fermenting the mixture to manufacture the fermented milk product.

2. The method according to claim 1, wherein forming the amino acid supplemented milk source comprises at least one of an addition of at least one amino acid, an addition of a protease or an addition of a peptidase to the milk source.

3. The method according to any one of the above claims further comprising drying the fermented milk product and forming a powder.

4. The method according to any one of the above claims further comprising concentrating the fermented milk product and forming a fermented milk product concentrate.

5. The method according to any one of the above claims, wherein the milk source is selected from at least one of full fat milk, skimmed milk, semi-skimmed milk, fresh milk, recombined milk, cream, buttermilk, whey and milk containing vegetable fat.

6. The method according to any one of the above claims, wherein the at least one amino acid is selected from L-phenylalanine, L-leucine, L-isoleucine, L-valine.

7. The method according to any one of the above claims, wherein the peptidase is an exo-peptidase.

8. The method according to any one of the above claims, wherein the protease is an exo-protease.

9. The method according to any one of the above claims further comprising an addition of at least one of a lipase enzyme and a lactase enzyme to the milk source.

10. The method according to any one of the above claims further comprising adding a fermentation co-factor to the milk source.

11. A fermented milk product with at least a malty flavour and aroma obtainable by the method of any of the above claims.

12. A product for consumption by a mammal comprising the fermented milk product of claim 11.

13. A food product comprising a Lactococcus lactis subsp. lactis biovar diacetylactis, deposited as CNCM I-1962, and any one of 2-methylpropanal, 2/3-methylbutanal, phenylacetaldehyde and 2/3-methylbutanol.

14. A use of a lactic acid bacteria Lactococcus lactis subsp. lactis biovar diacetylactis, deposited as CNCM I-1962, to impart at least a malty flavour and aroma to a milk source.

## Patentansprüche

1. Verfahren zur Herstellung eines fermentierten Milchprodukts mit mindestens einem Malzgeschmack und Aroma, umfassend:
- Bereitstellen einer Milchquelle,
- Bilden einer mit Aminosäure ergänzten Milchquelle,
- Zusetzen einer Lactococcus lactis subsp. lactis biovar diacetylactis, hinterlegt als CNCM I-1962, zu der mit Aminosäure ergänzten Milch, um ein Gemisch zu bilden; und
- Fermentieren der Mischung, um ein fermentiertes Milchprodukt herzustellen.

2. Verfahren nach Anspruch 1, wobei das Bilden der mit Aminosäure ergänzten Milchquelle mindestens eins von einem Zusetzen von mindestens einer Aminosäure, einem Zusetzen einer Protease oder einem Zusetzen einer Peptidase zu der Milchquelle umfasst.

3. Verfahren nach einem der vorstehenden Ansprüche, ferner umfassend Trocknen des fermentierten Milchprodukts und Bilden eines Pulvers.

4. Verfahren nach einem der vorstehenden Ansprüche, ferner umfassend Konzentrieren des fermentierten Milchprodukts und Bilden eines fermentierten Milchproduktkonzentrats.

5. Verfahren nach einem der vorstehenden Ansprüche, wobei die Milchquelle aus mindestens einem von Vollmilch, Magermilch, teilentrahmter Milch, frischer Milch, rekombinierter Milch, Sahne, Buttermilch, Molke und Milch, die pflanzliches Fett enthält, ausgewählt ist.

6. Verfahren nach einem der vorstehenden Ansprüche, wobei die mindestens eine Aminosäure ausgewählt ist aus L-Phenylalanin, L-Leucin, L-Isoleucin, L-Valin.

7. Verfahren nach einem der vorstehenden Ansprüche, wobei die Peptidase eine Exopeptidase ist.

8. Verfahren nach einem der vorstehenden Ansprüche, wobei die Protease eine Exoprotease ist.

9. Verfahren nach einem der vorstehenden Ansprüche, ferner umfassend ein Zusetzen von mindestens einem eines Lipaseenzyms und eines Lactaseenzyms zu der Milchquelle.

10. Verfahren nach einem der vorstehenden Ansprüche, ferner umfassend Zusetzen eines Fermentations-Kofaktors zu der Milchquelle.

11. Fermentiertes Milchprodukt mit mindestens einem Malzgeschmack und Aroma, die durch das Verfahren nach einem der vorstehenden Ansprüche erhältlich sind.

12. Produkt für den Verzehr durch einen Säuger, umfassend das fermentierte Milchprodukt gemäß Anspruch 11.

13. Lebensmittelprodukt, umfassend eine Lactococcus lactis subsp. lactis biovar diacetylactis, hinterlegt als CNCM I-1962, und beliebige von 2-Methylpropanal, 2/3-Methylbutanal, Phenylacetaldehyd und 2/3-Methylbutanol.

14. Verwendung einer Milchsäurebakterie Lactococcus lactis subsp. lactis biovar diacetylactis, hinterlegt als CNCM I-1962, um einer Milchquelle mindestens einen Malzgeschmack und Aroma zu verleihen.

## Revendications

1. Procédé de fabrication d'un produit laitier fermenté avec au moins un arôme et une saveur de malt comprenant :
- la fourniture d'une source de lait,
- la formation d'une source de lait enrichie en acide aminé,
- l'ajout, à la source de lait enrichie en acide aminé, d'un Lactococcus lactis sous-espèce lactis biovar diacetylactis, déposé à la CNCM sous le numéro 1-1962, afin de former un mélange ; et
- la fermentation du mélange pour fabriquer le produit laitier fermenté.

2. Procédé selon la revendication 1, dans lequel la formation de la source de lait enrichie en acide aminé comprend au moins une action parmi un ajout d'au moins un acide aminé, un ajout d'une protéase ou un ajout d'une peptidase à la source de lait.

3. Procédé selon l'une quelconque des revendications précédentes, comprenant en outre le séchage du produit laitier fermenté et la formation d'une poudre.

4. Procédé selon l'une quelconque des revendications précédentes, comprenant en outre la concentration du produit laitier fermenté et la formation d'un concentré de produit laitier fermenté.

5. Procédé selon l'une quelconque des revendications précédentes, dans lequel la source de lait est choisie parmi au moins une substance parmi le lait entier, le lait écrémé, le lait demi-écrémé, le lait frais, le lait recombiné, la crème, le babeurre, le lactosérum et du lait contenant de la matière grasse végétale.

6. Procédé selon l'une quelconque des revendications précédentes, dans lequel ledit au moins un acide aminé est choisi parmi la L-phénylalanine, la L-leucine, la L-isoleucine, la L-valine.

7. Procédé selon l'une quelconque des revendications précédentes, dans lequel la peptidase est une exo-peptidase.

8. Procédé selon l'une quelconque des revendications précédentes, dans lequel la protéase est une exo-protéase.

9. Procédé selon l'une quelconque des revendications précédentes, comprenant en outre un ajout d'au moins une parmi une enzyme de lipase et une enzyme de lactase à la source de lait.

10. Procédé selon l'une quelconque des revendications précédentes, comprenant en outre l'ajout d'un co-facteur de fermentation à la source de lait.

11. Produit laitier fermenté avec au moins un arôme et une saveur de malt, pouvant être obtenu par le procédé selon l'une quelconque des revendications précédentes.

12. Produit de consommation par un mammifère comprenant le produit laitier fermenté de la revendication 11.

13. Produit alimentaire comprenant un Lactococcus lactis sous-espèce lactis biovar diacetylactis, déposé à la CNCM sous le numéro 1-1962 et l'un quelconque parmi 2-méthylpropanal, 2/3-méthylbutanal, phénylacétaldéhyde et 2/3-méthylbutanol.

14. Utilisation de bactéries d'acide lactique Lactococcus lactis sous-espèce lactis biovar diacetylactis, déposées à la CNCM sous le numéro I-1962, afin de conférer au moins un arôme et une saveur de malt à une source de lait.
